# EUROPÄISCHE PATENTSCHRIFT

(11) **EP 2 160 156 B1**
(45) Veröffentlichungstag und Bekanntmachung des Hinweises auf die Patenterteilung: **15.06.2016**
(21) Anmeldenummer: 08773733.4
(22) Anmeldetag: 27.06.2008
(51) Int. Cl.: A61F 2/06, A61F 2/24

(54) **AORTENSINUSPROTHESE**
AORTIC SINUS PROSTHESIS
PROTHÈSE DE SINUS AORTIQUE

(30) Priorität: 27.06.2007 DE 102007031148
(43) Veröffentlichungstag der Anmeldung: 10.03.2010
(73) Patentinhaber: Aesculap AG, 78532 Tuttlingen/Donau (DE)
(72) Erfinder: GOLDMANN, Helmut, 34119 Kassel (DE); MERCKLE, Christof, 68199 Mannheim (DE); SIEVERS, Hans-Hinrich, 24119 Kronshagen (DE)
(74) Vertreter: Patentanwälte Ruff, Wilhelm, Beier, Dauster & Partner mbB
(86) Internationale Anmeldenummer: PCT/EP2008/005275
(87) Internationale Veröffentlichungsnummer: WO 2009/000546

(56) Entgegenhaltungen:
- WO-A-01/52776
- WO-A-90/14804
- WO-A-2005/034812
- WO-A1-2008/094894

## Beschreibung

Die Erfindung betrifft eine Aortensinusprothese mit einem im wesentlichen zylindrischen Prothesenabschnitt und einem daran angrenzenden im Vergleich zum zylindrischen Prothesenabschnitt erweiterten Bulbusabschnitt und gegebenenfalls einem dritten, im wesentlichen zylindrischen, herznahen Abschnitt, der sich an den Bulbusabschnitt anschließt.

Bei bekannten Aortensinusprothesen kann der Bulbusabschnitt in Form eines im Umfang gleichmäßig aufgeweiteten ballonartigen Abschnittes ausgebildet sein, wie dies beispielsweise in der EP 0 955 019 B1 beschrieben ist. Der Bulbusabschnitt kann eine Plissierung aufweisen, die längs oder quer verlaufen kann. Ein derartiger Bulbusabschnitt ist zwar in der Herstellung relativ einfach. Er entspricht jedoch nicht den natürlichen Verhältnissen der Aortenwurzel und der Aortenklappe. Die Aortenklappe hat normalerweise drei Segel, die an drei vertikalen Kommissuren aufgehängt sind. Zwischen diesen Kommissuren besitzt die Aortenwand Ausbuchtungen, die Sinus genannt werden und den Segeln der Herzklappen genügend radialen Spielraum gewähren. Bei einer Aortensinusprothese mit einem rotationssymmetrischen Bulbus haben die Segel zwar den normalen radialen Spielraum, die Kommissuren finden aber durch die Prothese im Bereich der Kommissuren keine Unterstützung.

Es gibt auch Aortensinusprothesen, die im Bereich der Aortenwurzel anstelle eines rotationssymmetrischen Bulbus drei getrennte Sinus aufweisen. Solche Sinus werden in der Regel als Einzelstücke in entsprechende Ausschnitte in der Wandung der Aortensinusprothese eingesetzt. Abgesehen davon, dass die Herstellungsweise einer solchen Aortensinusprothese relativ aufwendig ist, sind ihre Einsatzmöglichkeiten auch begrenzt. Denn es kommt immer wieder vor, dass eine Herzklappe asymmetrisch ausgebildet ist. So kann ein Segel einer Herzklappe deutlich kleiner, insbesondere schmaler sein als die beiden anderen. Im Extremfall kann es sogar vorkommen, dass eine Herzklappe nur zwei Segel betrifft. Die Aortensinusprothese sollte dem Rechnung tragen können.

Die WO 01/52776 A1 bietet eine Lösung dieses Problems, indem einzelne Sinusflicken in Abhängigkeit von den Verhältnissen beim Patienten in der gewünschten Größe aus Gefäßprothesenwandungsmaterial zugeschnitten und einzeln an eine Aortensinuswurzelprothese angenäht werden. Ein solches Vorgehen ist jedoch außerordentlich zeitaufwendig.

Aus der WO 90/14804 A1 ist eine Gefäßprothese mit einer Stützkäfigstruktur bekannt.

Der Erfindung liegt die Aufgabe zugrunde, eine Aortensinusprothese zur Verfügung zu stellen, die es dem Chirurgen ermöglicht, während der Operation in einfacher Weise eine Anpassung an die anatomischen Verhältnisse vorzunehmen, wobei eine Unterstützung der Kommissuren an der richtigen Stelle am Umfang der Prothese gewährleistet ist.

Diese Aufgabe wird dadurch gelöst, dass bei einer Aortensinusprothese der eingangs erwähnten Art dem Bulbusabschnitt zur Unterteilung des Bulbus in mindestens zwei Sinus und zur Unterstützung der Kommissuren der Herzklappe mindestens zwei Längsstege zugeordnet sind, die an der Außenseite des Bulbusabschnittes positionierbar und fixierbar sind.

Normalerweise handelt es sich um drei Stege entsprechend den drei Kommissuren der Herzklappe. Im Falle der Anomalie der Aortenklappe mit nur zwei Segeln reichen zwei Längsstege aus. Die Längsstege haben die Aufgabe, dem Bulbusabschnitt im Bereich der Kommissuren der Herzklappe eine Unterstützung gegen eine radiale Aufweitung zu geben, so dass sich zwischen den Längsstegen Ausbuchtungen ergeben, die den natürlichen Sinus entsprechen, und der Durchmesser der Aortensinusprothese im Bereich der Stege in etwa den im wesentlichen zylindrischen Prothesenabschnitten unter- und oberhalb des Bulbusbereich, entspricht.

Bei einer Ausführungsform der Erfindung ist es vorgesehen, dass die Längsstege getrennt von der Prothese vorliegen und an dieser an den geeigneten Stellen befestigbar sind. Die Länge der Längsstege entspricht im Wesentlichen der Länge des Bulbusabschnittes. Vorzugsweise ist sie etwas größer, damit die Längsstege an dem oder den zylindrischen Prothesenabschnitten befestigbar sind. Normalerweise reicht eine Befestigung der Längsstege an ihrem oberen und unteren Ende aus, so dass sie im Zwischenbereich ohne Befestigung auf dem Bulbusabschnitt aufliegen. Sie können aber auch beim Einnähen der Kommissuren der natürlichen Herzklappe mit in die Naht eingefasst sein. Es ist auch möglich, die Längsstege etwas kürzer zu halten als der Entfernung der Befestigungsstellen entspricht. Dadurch kann eine Raffung des Bulbus im Bereich der Längsstege erzielt werden, was die Ausbildung der Sinus begünstigt.

Die mindestens zwei Längsstege, insbesondere drei Längsstege, sind einzeln, d.h. unabhängig voneinander, positionierbar.

Bei einer weitergehenden Ausführungsform der Erfindung ist ein Längssteg an der Prothese bereits ortsfest befestigt. Der mindestens eine andere Längssteg, insbesondere die beiden anderen Längsstege sind an gewünschter Stelle, vorzugsweise unabhängig voneinader, positionierbar. Diese Ausführungsform ist dann besonders günstig, wenn die Aortensinusprothese geradlinig verläuft, d.h. am herzfernen Ende nicht die vorfixierte Form eines Aortenbogens ausgebildet ist. Bei geradlinigem Verlauf kann die Prothese ohne Probleme um die eigene Achse gedreht werden, so dass der vorab befestigte Längssteg in Übereinstimmung mit einer Kommissur positionierbar ist.

Bei einer weiteren Ausführungsform, bei denen die Aortensinusprothese zusätzlich die Form eines Aortenbogens aufweist bzw. mit einem solchen verbunden ist, sind vorzugsweise alle Längsstege im wesentlichen frei, d.h. unabhängig voneinander, am Umfang des Bulbus positionierbar, weil es hier zweckmäßig ist, die Lage der Aortensinusprothese in Abhängigkeit vom Verlauf des Aortenbogens auszurichten.

Bei einer weiteren Ausführungsform der Erfindung ist mindestens ein Steg, sind vorzugsweise mindestens zwei Stege und gegebenenfalls alle drei Stege am Außenumfang des Bulbus parallel zur Längsachse der Prothese verschiebbar angeordnet, insbesondere geführt. Dies bedeutet, dass die entsprechende Anzahl von Stegen an der Aortensinusprothese im Bereich des Bulbus befestigt aber parallel zur Längsachse der Aortensinusprothese am Außenumfang des Bulbus verschiebbar ist. Eine solche Vorfixierung der Stege erleichtert die Arbeit des Chirurgen, da dieser die relative Anordnung der Stege zueinander in einfacher Weise vornehmen kann und es dann lediglich noch erforderlich ist, die Stege in der jeweiligen Position festzulegen bzw. zu fixieren. Auch hier ist es wiederum möglich, einen Steg bereits ortsfest vorzusehen, so dass lediglich noch der mindestens eine weitere an der richtigen Stelle zu fixieren ist.

Zur Führung der Längsstege können mindestens zwei schienenartige Führungselemente in einem axialen Abstand voneinander um mindestens einen Teil des Prothesenumfangs am Außenumfang der Prothese angeordnet sein. Die schienenartigen Führungselemente sind vorzugsweise im Bereich der Enden des Bulbusabschnittes vorgesehen, insbesondere am Übergang von den zylindrischen Abschnitten in den Bulbusabschnitt angeordnet. Der mindestens eine positionierbare, insbesondere die mindestens zwei positionierbaren Längsstege können mit ihren oberen und unteren Enden mit den Führungselementen verbunden sein. Eine verschiebbare Verbindung ist bevorzugt.

Die Führungselemente können von Schnüren und dergleichen gebildet sein, wobei beispielsweise auch Drähte und Bänder in Frage kommen. Die Führungselemente sind vorzugsweise zwischen jeweils zwei Positionierungsstellen der Stege an der Prothese befestigt. Die Befestigung kann eine Fixierung sein. Es ist auch möglich, die Führungselemente entlang des Prothesenumfangs verschiebbar zu befestigen. Bei dieser Ausführungsform ist es möglich, die Längsstege mit ihren oberen und unteren Enden an den Führungselementen unverschieblich zu befestigen, so dass die Einstellung der Positionierung durch Drehung der verschiebbaren Führungselemente am Prothesenumfang vorgenommen wird. Schließlich ist es auch möglich, sowohl die Längsstege auf den Führungselementen verschiebbar zu führen als auch die Führungselemente am Prothesenumfang verschiebbar zu befestigen.

Die Längsstege können aus textilem Material bestehen, wodurch ein Fixieren der Stege in der richtigen Position durch Annähen einfach möglich ist. Die Längsstege können vorzugsweise auch eine Versteifung aufweisen. Diese Versteifung kann elastisch ausgebildet sein. So können Versteifungselemente auf die textilen Stege auf- oder eingenäht sein. Als Versteifungselemente eignen sich monofile Drähte, insbesondere solche aus Metall.

Offenbart ist auch ein Kit für eine Aortensinusprothese mit mindestens einem im wesentlichen zylindrischen Prothesenabschnitt und einem daran angrenzenden im Vergleich zum Prothesenabschnitt erweiterten Bulbusabschnitt und gegebenenfalls einem herznahen sich an den Bulbusabschnitt anschließenden weiteren zylindrischen Abschnitt. Der Kit umfasst mindestens zwei, vorzugsweise drei, Längsstege, die am Außenumfang des Bulbus positionierbar und befestigbar ausgebildet sind.

Die Aortensinusprothese kann textiler oder nicht textiler Natur sein oder aus einer Kombination von beiden bestehen. Sie kann aus einem Stück sein oder aus zum Beispiel drei Stücken zusammengesetzt sein. Bevorzugte Materialien sind Polyester, insbesondere Polyethylenterephthalat, Polytetrafluorethylen, insbesondere expandiertes Polytetrafluorethylen oder Polyurethane, insbesondere aliphatische Polyurethane.

Weitere Merkmale der Erfindung ergeben sich aus der nachfolgenden Beschreibung von bevorzugten Ausführungsformen in Verbindung mit den Zeichnungen und den Unteransprüchen. Hierbei können die einzelnen Merkmale jeweils für sich alleine oder zu mehreren bei einer Ausführungsform der Erfindung verwirklicht sein.

In den Zeichnungen zeigen
- Figur 1a:: eine Aortensinusprothese,
- Figur 1b:: Längsstege, die der Aortensinusprothese nach Figur 1a zugeordnet sind,
- Figur 2:: eine weiteren Ausführungsform,
- Figur 3:: einen Schnitt entlang der Linie III-III nach Figur 2,
- Figur 4:: eine weitere Ausführungsform und
- Figur 5:: eine weitere Ausführungsform.

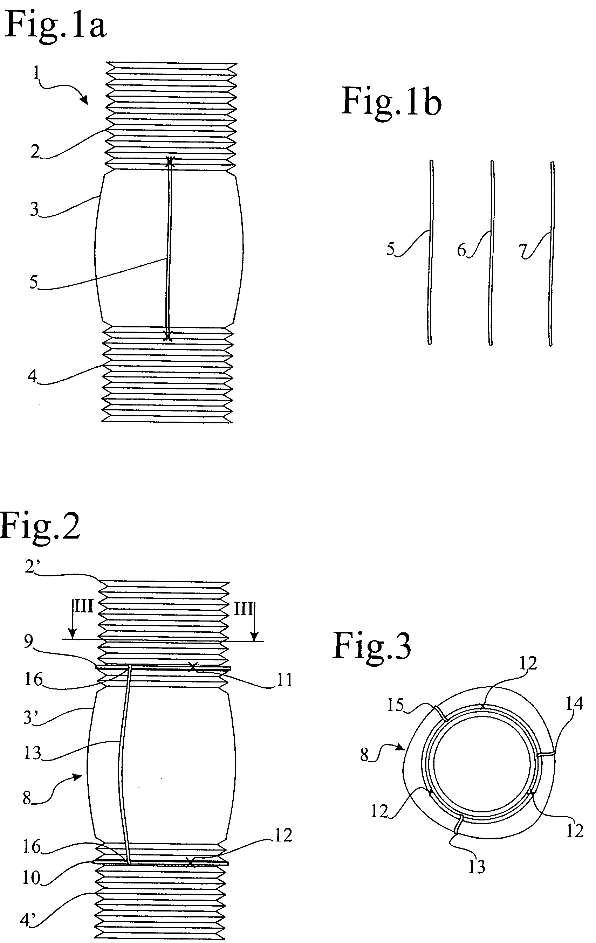

Bei der in der Zeichnug in Figuren 1a und 1b dargestellten Ausführungsform ist eine Aortensinusprothese 1 in Seitenansicht dargestellt. Diese weist einen oberen (herzfernen) im wesentlichen zylindrischen Prothesenabschnitt 2 auf. An dessen unteren Ende schließt sich ein ursprünglich rotationssymmetrischer Bulbusabschnitt 3 an, der eine ballonähnliche Form besitzt. Der Bulbusabschnitt 3 kann plissiert sein, beispielsweise eine Querplissierung oder eine Längsplissierung aufweisen. Am unteren Ende des Bulbusabschnittes 3 schließt sich ein herznaher zylindrischer Abschnitt 4 an. Dieser kann sehr kurz gehalten oder kürzbar ausgebildet sein und ebenfalls plissiert, insbesondere aber unplissiert sein. Die Prothese besteht vorzugsweise aus textilem Material. Sie kann gewebt oder gewirkt sein. Dem Bulbusabschnitt 4 sind drei Längsstege 5, 6 und 7 zugeordnet. Diese Stege bestehen aus textilem Material und können mit einer Versteifung versehen sein, beispielsweise in Form eines eingenähten Kunststoffdrahtes. Die Längsstege haben etwa die Länge des Bulbusabschnittes oder sind geringfügig länger. Die Längsstege 5, 6 und 7 sind an der Außenseite des Bulbusabschnittes befestigbar. Die Befestigung erfolgt vorzugsweise lediglich am oberen und unteren Ende der Längsstege und zwar insbesondere an den oberen und unteren Enden des Bulbusabschnittes bzw. an den angrenzenden Enden der zylindrischen Abschnitte. Die Befestigung kann durch einfaches Annähen erfolgen.

Je nach dem, ob die Aortenklappe drei Segel aufweist, was der Normalfall ist, oder nur zwei Segel, was die Ausnahme ist, werden zwei oder drei Längsstege am Außenumfang des Bulbusabschnittes positioniert. Da bei zwei Stegen nur ein Steg frei positionierbar zu sein braucht und bei drei Stegen nur zwei Stege, kann ein Steg bereits bei der Herstellung der Aortensinusprothese im Bereich des Bulbus fixiert werden, wie dies in Figur 1a mit dem Längssteg 5 dargestellt ist. Die Freiheitsgrade, die durch die Größe und die Zahl der Segel und der dazwischen liegenden Kommissuren bestimmt werden, sind dann durch die restlichen Stege bedienbar. Der Durchmesser der zylindrischen Abschnitte 2 und 4 kann gleich groß sein. Vorzugsweise hat der herznahe zylindrische Abschnitt einen etwas größeren Durchmeser. Eine solche Ausbildung ist auch bei den weiteren Ausführungsformen möglich.

Bei der Ausführungsform nach den Figuren 2 und 3 sind drei Längsstege am Außenumfang des Bulbusabschnittes in Umfangsrichtung parallel zur Längsachse der Aortensinusprothese verschiebbar befestigt. Die Aortensinusprothese 8 weist wiederum einen oberen zylindrischen Abschnitt 2', einen Bulbusabschnitt 3' und einen unteren herznahen zylindrischen Abschnitt 4' auf. Zur Führung der Längsstege sind am oberen und unteren Ende des Bulbusabschnittes 3' zwei Bändchen 9 und 10 um die zylindrichen Abschnitte 2' und 4' der Prothese gelegt und an jeweils bis zu drei oberen Fixpunkten 11 und bis zu drei unteren Fixpunkten 12 mit der Prothese fest verbunden. Die Längsstege 13, 14 und 15 besitzen an ihren oberen und unteren Enden Schlaufen 16, durch die sie auf den Bändchen 9 und 10 in Umfangsrichtung des Bulbus 3' parallel zu Längsachse der Prothese verschiebbar geführt sind. Die Längsstege sind dabei einzeln, d.h. unabhängig voneinander, verschiebbar. Wie aus Figur 3 ersichtlich, ist jedes Bändchen um 120 ° in Umfangsrichtung verschiebbar. Dadurch können die Längsstege den tatsächlichen Verhältnissen einer Herzklappe angepasst werden. Es ist sogar möglich, zwei Bändchen in einem Winkel von 180 ° zueinander auszurichten und das dritte Bändchen zu entfernen, so dass diese Prothese auch für eine Herzklappe mit nur zwei Segeln verwendbar ist. Weiterhin ist aus Figur 3 ersichtlich, dass die Längsstege eine Einschnürung des Bulbusabschnittes 3' in Längsrichtung bewirken. Diese Einschnürung dient zur Ausbildung der Sinus und zur Abstützung der Kommissuren der Herzklappe.

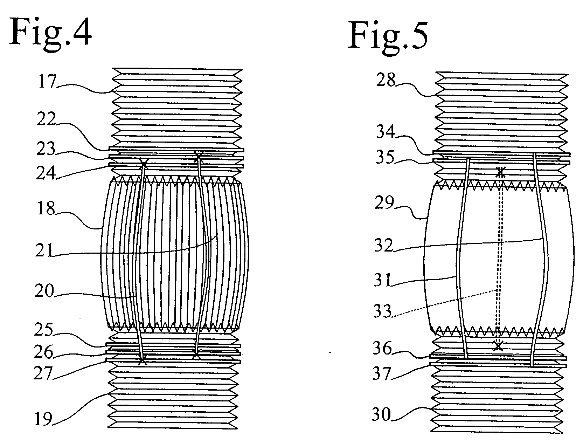

Bei der Ausführungsform nach Figur 4 weist eine Aortensinusprothese wiederum einen oberen zylindrischen Abschnitt 17, einen Bulbusabschnitt 18 und einen unteren zylindrischen Abschnitt 19 auf. Es sind wiederum drei Längsstege vorgesehen, von denen auf der Vorderseite nur die Stege 20 und 21 sichtbar sind. Zur verschiebbaren Befestigung der Stege sind jedem Steg zwei Bändchen zugeordnet, nämlich jeweils ein oberes und ein unteres, so dass insgesamt drei obere Bändchen 22, 23, 24 und drei untere Bändchen 25, 26 und 27 vorgesehen sind. Die Führungsbändchen sind um die Aortensinusprothese drehbar angeordnet. Die Längsstege sind auf den jeweiligen Bändchen fixiert, so dass die geeignete Positionierung der Längsstege durch Drehen der Bändchen mit den Stegen einstellbar ist. Bei der Ausführungsform nach Figur 4 sind die Längsstege 21, 22 und auch der dritte nicht sichtbare Längssteg gleich lang. Dementsprechend ist der Steg 20 mit seinem oberen Ende am oberen Bändchen 24 befestigt und an seinem unteren Ende mit dem unteren Bändchen 27. Die weiteren Stege entsprechend nach oben abgestaffelt.

Bei der Ausführungsform nach Figur 5 ist wiederum ein oberer zylindrischer Prothesenabschnitt 28 vorgesehen. Es schließt sich ein Bulbusabschnitt 29 an und daran wiederum ein herznaher zylindrischer Abschnitt 30. Es sind wiederum drei Längsstege 31, 32 und 33 vorgesehen, wobei sich der Längssteg 33 auf der Rückseite des Bulbusabschnittes befindet. Dieser Längssteg ist bereits mit seinen oberen und unteren Enden an der Prothese fixiert, d.h. nicht mehr verschieblich. Die beiden anderen Stege 31 und 32 sind vorzugsweise unabhängig voneinander verschiebbar angeordnet. Hierzu sind wiederum umlaufende obere Bändchen 34 und 35 und untere Bändchen 36 und 37 vorgesehen. Im Unterschied zur Ausführungsform nach Figur 4 sind die Längsstege 31 und 32 verschieden lang. Der Längssteg 31 ist kürzer und mit seinem oberen und unteren Ende am bulbusnahen oberen Bändchen 35 und bulbusnahen unteren Bändchen 36 befestigt. Der längere Längssteg 32 ist am bulbusferneren oberen Bändchen 34 und am bulbusferneren unteren Bändchen 37 befestigt. Bei dieser Ausführungsform ist es möglich, den Längssteg 32 mit den Führungsbändchen über den Längssteg 31 und den Längssteg 33 zu schieben. Dadurch wird eine noch bessere Anpassung an die anatomischen Verhältnisse ermöglicht. So kann beispielsweise ein Längssteg 31 steifer sein als ein Längssteg 32, so dass verschieden starke Längseinschnürungen des Bulbus an beliebiger Stelle der Aortensinusprothese möglich sind.

Bei den Ausführungsformen nach den Figuren 4 und 5 ist es wiederum möglich, einen Steg zu entfernen, falls lediglich zwei Stege erforderlich sind. Bei den Ausführungsformen nach den Figuren 3 bis 5 ist es auch möglich, diese Ausführungsformen so auszugestalten, dass sowohl die Längsstege relativ zu den Führungsbändchen bzw. Führungselementen verschiebbar sind als auch die Führungselemente relativ zur Aortensinusprothese. Dadurch werden besonders große Freiheitsgrade geschaffen. Die endgültige Fixierung erfolgt dann jeweils durch Aufheben der relativen Verschiebbarkeit, was vorzugsweise durch einfaches Annähen der relativ zueinander verschiebbaren Elemente erfolgt. Wenn die Fixierung der Längsstege unmittelbar an der Aortensinusprothese vorgenommen wird, können die Führungselemente entfernt werden, da sie dann keine Funktion mehr ausfüllen.

## Patentansprüche

1. Aortensinusprothese mit mindestens einem im wesentlichen zylindrischen Prothesenabschnitt (2, 4; 2', 4'; 17, 19; 28, 30) und einem daran angrenzenden im Vergleich zum zylindrischen Prothesenabschnitt erweiterten Bulbusabschnitt (3; 3'; 18; 29), wobei dem Bulbusabschnitt zur Unterteilung des Bulbus in mindestens zwei Sinus und zur Unterstützung der Kommissuren der Herzklappe mindestens zwei Längsstege (5, 6, 7; 13, 14, 15; 20, 21; 31, 32, 33) zugeordnet sind, die an der Außenseite des Bulbusabschnittes positionierbar und fixierbar sind, **dadurch gekennzeichnet, dass** die mindestens zwei Längsstege (5, 6, 7, 13, 14, 15, 20, 21, 31, 32, 33) einzeln, d.h. unabhängig voneinander, positionierbar sind.

2. Aortensinusprothese nach Anspruch 1, **dadurch gekennzeichnet, dass** die Längsstege (5, 6, 7; 13, 14, 15; 20, 21; 31, 32, 33) am Außenumfang des Bulbus (3; 3'; 18; 29) entsprechend der tatsächlichen symmetrischen oder asymmetrischen Ausbildung der Herzklappe eines Patienten im wesentlichen parallel zur Prothesenlängsachse positionierbar ausgebildet sind.

3. Aortensinusprothese nach Anspruch 1 oder 2, **dadurch gekennzeichnet, dass** die Längsstege (5, 6, 7) getrennt von der Prothese vorliegen und an dieser befestigbar sind.

4. Aortensinusprothese nach einem der vorhergehenden Ansprüche, **dadurch gekennzeichnet, dass** ein Längssteg (5; 33) an der Prothese ortsfest befestigt ist und der mindestens eine andere Längssteg, insbesondere die beiden anderen Längsstege (6, 7; 31, 32), an gewünschter Stelle positionierbar sind.

5. Aortensinusprothese nach einem der vorhergehenden Ansprüche, **dadurch gekennzeichnet, dass** mindestens ein, insbesondere zwei Stege (13, 14, 15; 20, 21; 31, 32) am Außenumfang des Bulbus (3'; 18; 29) parallel zur Längsachse der Prothese verschiebbar angeordnet, insbesondere geführt sind.

6. Aortensinusprothese nach einem der vorhergehenden Ansprüche, **dadurch gekennzeichnet, dass** mindestens zwei schienenartige Führungselemente (9, 10; 22, 23, 24, 25, 26, 27; 34, 35, 36, 37) in einem axialen Abstand um mindestens einen Teil des Prothesenumfangs am Außenumfang der Prothese angeordnet sind.

7. Aortensinusprothese nach Anspruch6, **dadurch gekennzeichnet, dass** die schienenartigen Führungselemente (9, 10; 22, 23, 24, 25, 26, 27; 34, 35, 36, 37) im Bereich der Enden des Bulbusabschnittes vorgesehen sind.

8. Aortensinusprothese nach Anspruch 6 oder7, **dadurch gekennzeichnet, dass** der mindestens eine positionierbare, insbesondere die mindestens zwei Längsstege (13, 14, 15; 20, 21; 31, 32) mit ihren oberen und unteren Enden mit den Führungselementen (9, 10; 22, 23, 24, 25, 26, 27; 34, 35, 36, 37), insbesondere verschiebbar, verbunden sind.

9. Aortensinusprothese nach einem der Ansprüche 6 bis8, **dadurch gekennzeichnet, dass** die vorzugsweise von Schnüren gebildeten Führungselemente (22, 23, 24, 25, 26, 27; 34, 35, 36, 37) zwischen jeweils zwei Positionierungsstellen der Stege an der Prothese, insbesondere verschiebbar, befestigt sind.

10. Aortensinusprothese nach einem der Ansprüche 6 bis9, **dadurch gekennzeichnet, dass** für jeden verschiebbaren Steg (20, 21; 31, 32) getrennte Führungselemente (22, 23, 24, 25, 26, 27; 34, 35, 36, 37) vorgesehen sind und die Führungselemente am Außenumfang der Prothese verschiebbar ausgebildet sind, wobei die Längsstege an den Führungselementen unverschiebbar oder verschiebbar befestigt sind.

11. Aortensinusprothese nach einem der vorhergehenden Ansprüche, **dadurch gekennzeichnet, dass** die Längsstege (5, 6, 7; 13, 14, 15; 20, 21; 31, 32, 33) aus textilem Material bestehen.

12. Aortensinusprothese nach einem der vorhergehenden Ansprüche, **dadurch gekennzeichnet, dass** die Längsstege (5, 6, 7) elastisch versteift sind, insbesondere durch monofile Drähte verstärkt sind.

## Claims

1. Aortic sinus prosthesis, comprising at least one essentially cylindrical prosthesis segment (2, 4; 2', 4'; 17, 19; 28, 30) and adjoining thereto a bulbus segment (3; 3'; 18; 29), which is enlarged as compared to the cylindrical prosthesis segment, the bulbus segment being assigned at least two longitudinal links (5, 6, 7; 13, 14, 15; 20, 21; 31, 32, 33) for subdividing the bulbus into at least two sinuses and for supporting the commissures of the cardiac valve, which links are positionable and fixable on the exterior side of the bulbus segment,
**characterized in that**
the at least two longitudinal links (5, 6, 7, 13, 14, 15, 20, 21, 31, 32, 33) are positionable distinctly, i.e., one independently from the other.

2. Aortic sinus prosthesis according to claim 1, **characterized in that** the longitudinal links (5, 6, 7; 13, 14, 15; 20, 21; 31, 32, 33) are configured on the exterior circumference of the bulbus (3; 3'; 18; 29) corresponding to the actual symmetrical or asymmetrical feature of the cardiac valve of a patient to be positionable essentially in parallel to the longitudinal axis of the prosthesis.

3. Aortic sinus prosthesis according to claim 1 or 2, **characterized in that** the longitudinal links (5, 6, 7) are provided separate from the prosthesis and are attachable thereto.

4. Aortic sinus prosthesis according to any one of the preceding claims, **characterized in that** one longitudinal link (5; 33) is attached to the prosthesis fixed in position and the at least one other longitudinal link, in particular the two other longitudinal links (6, 7; 31, 32), are positionable at a desired location.

5. Aortic sinus prosthesis according to any one of the preceding claims, **characterized in that** at least one, in particular two links (13, 14, 15; 20, 21; 31, 32) are disposed, in particular are guided, on the exterior circumference of the bulbus (3'; 18; 29) to be displaceable in parallel to the longitudinal axis of the prosthesis.

6. Aortic sinus prosthesis according to any one of the preceding claims, **characterized in that** at least two rail-type guiding elements (9, 10; 22, 23, 24, 25, 26, 27; 34, 35, 36, 37) are disposed on the exterior circumference of the prosthesis in an axial distance about at least part of the circumference of the prosthesis.

7. Aortic sinus prosthesis according to claim 6, **characterized in that** the rail-type guiding elements (9, 10; 22, 23, 24, 25, 26, 27; 34, 35, 36, 37) are provided in the vicinity of the ends of the bulbus segment.

8. Aortic sinus prosthesis according to claim 6 or 7, **characterized in that** the at least one positionable, in particular the at least two longitudinal links (13, 14, 15; 20, 21; 31, 32) are connected with their upper and lower ends to the guiding elements (9, 10; 22, 23, 24, 25, 26, 27; 34, 35, 36, 37), in particular in a displaceable manner.

9. Aortic sinus prosthesis according to any one of claims 6 to 8, **characterized in that** the guiding elements (22, 23, 24, 25, 26, 27; 34, 35, 36, 37), preferably composed of strings, are attached between in each case two positioning locations of the links on the prosthesis, in particular in a displaceable manner.

10. Aortic sinus prosthesis according to any one of claims 6 to 9, **characterized in that** for each displaceable link (20, 21; 31, 32) separate guiding elements (22, 23, 24, 25, 26, 27; 34, 35, 36, 37) are provided and the guiding elements are disposed on the exterior circumference of the prosthesis to be displaceable, wherein the longitudinal links are attached to the guiding elements to be non-displaceable or displaceable.

11. Aortic sinus prosthesis according to any one of the preceding claims, **characterized in that** the longitudinal links (5, 6, 7; 13, 14, 15; 20, 21; 31, 32, 33) are composed of textile material.

12. Aortic sinus prosthesis according to any one of the preceding claims, **characterized in that** the longitudinal links (5, 6, 7) are elastically stiffened, in particular reinforced using monofilament wires.

## Revendications

1. Prothèse de sinus aortique comprenant au moins un tronçon de prothèse essentiellement cylindrique (2, 4 ; 2', 4' ; 17, 19 ; 28, 30) et un tronçon de bulbe (3, 3' ; 18 ; 29) adjacent à celui-ci, élargi comparé au tronçon de prothèse cylindrique, sachant qu'au moins deux branches longitudinales (5, 6, 7 ; 13, 14, 15 ; 20, 21 ; 31, 32, 33) sont attribuées au tronçon de bulbe pour subdiviser le bulbe en au moins deux sinus et pour soutenir les commissures de la valvule, lesquelles peuvent être positionnées et fixées sur le côté extérieur du tronçon de bulbe, **caractérisée en ce que** les au moins deux branches longitudinales (5, 6, 7 ; 13, 14, 15 ; 20, 21 ; 31, 32, 33) peuvent être positionnées individuellement, c'est à dire indépendamment l'une de l'autre.

2. Prothèse de sinus aortique selon la revendication 1, **caractérisée en ce que** les branches longitudinales (5, 6, 7 ; 13, 14, 15 ; 20, 21 ; 31, 32, 33) sont formées en pouvant être positionnées essentiellement parallèlement à l'axe longitudinal de la prothèse sur le pourtour extérieur du bulbe (3, 3' ; 18 ; 29) conformément à la conformation réelle symétrique ou asymétrique de la valvule d'un patient.

3. Prothèse de sinus aortique selon la revendication 1 ou 2, **caractérisée en ce que** les branches longitudinales (5, 6, 7) sont présentées séparées de la prothèse et peuvent être fixées sur celle-ci.

4. Prothèse de sinus aortique selon l'une des revendications précédentes, **caractérisée en ce qu'**une branche longitudinale (5 ; 33) est attachée fixement sur la prothèse et l'au moins une autre branche longitudinale, en particulier les deux autres branches longitudinales (6, 7 ; 31, 32) peuvent être positionnées à l'endroit souhaité.

5. Prothèse de sinus aortique selon l'une des revendications précédentes, **caractérisée en ce qu'**au moins une, en particulier deux, branche(s) (13, 14, 15 ; 20, 21 ; 31, 32) est/sont disposée(s) en étant mobile(s), en particulier dirigée(s), parallèlement à l'axe longitudinal de la prothèse sur le pourtour extérieur du bulbe (3' ; 18 ; 29).

6. Prothèse de sinus aortique selon l'une des revendications précédentes, **caractérisée en ce qu'**au moins deux éléments de guidage de type rail (9, 10 ; 22, 23, 24, 25, 26, 27 ; 34, 35, 36, 37) sont disposés à une distance axiale autour d'au moins une partie du pourtour de la prothèse sur le pourtour extérieur de la prothèse.

7. Prothèse de sinus aortique selon la revendication 6, **caractérisée en ce que** les éléments de guidage de type rail (9, 10 ; 22, 23, 24, 25, 26, 27 ; 34, 35, 36, 37) sont prévus au niveau des extrémités du tronçon de bulbe.

8. Prothèse de sinus aortique selon la revendication 6 ou 7, **caractérisée en ce que** l'au moins une, en particulier les au moins deux, branche(s) longitudinale(s) (13, 14, 15 ; 20, 21 ; 31, 32) positionnable(s) est/sont reliée(s), en particulier de façon mobile, aux éléments de guidage (9, 10; 22, 23, 24, 25, 26, 27 ; 34, 35, 36, 37) par ses/leurs extrémités supérieure(s) et inférieure(s).

9. Prothèse de sinus aortique selon l'une des revendications 6 à 8, **caractérisée en ce que** les éléments de guidage (22, 23, 24, 25, 26, 27 ; 34, 35, 36, 37) formés de préférence par des cordons sont fixés, en particulier de façon mobile, entre aux moins deux emplacements de positionnement des branches sur la prothèse.

10. Prothèse de sinus aortique selon l'une des revendications 6 à 9, **caractérisée en ce que** des éléments de guidage séparés (22, 23, 24, 25, 26, 27 ; 34, 35, 36, 37) sont prévus pour chaque branche mobile (20, 21 ; 31, 32) et les éléments de guidage sont formés en étant mobiles sur le pourtour extérieur de la prothèse, sachant que les branches longitudinales sont fixées sur les éléments de guidage en étant immobiles ou mobiles.

11. Prothèse de sinus aortique selon l'une des revendications précédentes, **caractérisée en ce que** les branches longitudinales (5, 6, 7 ; 13, 14, 15 ; 20, 21 ; 31, 32, 33) sont en matériau textile.

12. Prothèse de sinus aortique selon l'une des revendications précédentes, **caractérisée en ce que** les branches longitudinales (5, 6, 7) sont renforcées de manière élastique, en particulier par des fils monofibre.
